(19) Europäisches Patentamt
European Patent Office
Office européen des brevets

(11) Numéro de publication: **0 239 436 B1**

(12) # FASCICULE DE BREVET EUROPEEN

(45) Date de publication du fascicule du brevet: 20.06.90

(51) Int. Cl.[5]: **C07D 281/02**, A61K 31/55

(21) Numéro de dépôt: 87400371.8

(22) Date de dépôt: 20.02.87

(54) **Nouveau dérivé tricyclique dénommé acide (chloro-3 méthyl-6 dioxo-5,5 dihydro-6, 11 dibenzo (c,f) thiazépine (1,2) yl-11 amino) -5 pentanoique, son procédé de préparation et les compositions pharmaceutiques qui le contiennent.**

(30) Priorité: 21.02.86 FR 8602399

(43) Date de publication de la demande: 30.09.87 Bulletin 87/40

(45) Mention de la délivrance du brevet: 20.06.90 Bulletin 90/25

(84) Etats contractants désignés: AT BE CH DE FR GB IT LI LU NL SE

(56) Documents cités:
FR-A-2 104 728

JOURNAL OF CHROMATOGRAPHY, vol. 381, no. 1, 22 août 1986, pages 115-126, Amsterdam, NL; G. NICOT et al.: "Ion-pair extraction and high-performance liquid chromatographic determination of tianeptine and its metabolites in human plasma, urine and tissues"2-a]pyrimidin-5-ones"NDEX, CHEMICAL SUBSTANCES, page 52194cs, right-hand column, 3rd compound 000

(73) Titulaire: ADIR ET COMPAGNIE, 22, rue Garnier, F-92200 Neuilly-sur-Seine(FR)

(72) Inventeur: Malen, Charles, 3 allée Traversière, F-94260 Fresnes(FR)
Inventeur: Poignant, Jean-Claude, La Guyonnerie 13, rue de la Fontaine St-Mathieu, F-91440 Bures sur Yvette(FR)

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 239 436 B1

ACTORUM AG

## Description

La présente invention concerne un nouveau dérivé tricyclique, l'acide [(chloro-3 méthyl-6 dioxo-5,5 dihydro-6,11 dibenzo (c,f) thiazépine (1,2) yl-11) amino]-5 pentanoïque, son procédé de préparation et les compositions pharmaceutiques le renfermant.

Certains acides tricycliques dérivés de la (méthyl-6 dioxo-5,5 dihydro-6,11 dibenzo (c,f) thiazépine (1,2) yl-11) amine possédant d'intéressantes propriétés pharmacologiques ont été décrits dans le brevet français N°71.32197. Parmi ces produits, l'acide [(chloro-3 méthyl-6 dioxo-5,5 dihydro-6,11 dibenzo (c,f) thiazépine (1,2) yl-11) amino]-7 heptanoïque s'est distingué pour ses propriétés antidépressives et est utilisé de ce fait dans le traitement de la dépression.

Compte tenu des résultats thérapeutiques très intéressants obtenus sur ce produit, des recherches plus approfondies ont été entreprises dans la même série chimique et ont abouti à la découverte de l'acide [(chloro-3 méthyl-6 dioxo-5,5 dihydro-6,11 dibenzo (c,f) thiazépine (1,2) yl-11) amino]-5 pentanoïque, objet de la présente invention qui possède un profil antidépresseur original, d'intensité largement supérieure à celle des produits décrits dans le brevet mentionné ci-dessus.

La présente invention a plus particulièrement pour objet l'acide [(chloro-3 méthyl-6 dioxo-5,5 dihydro-6,11 dibenzo (c,f) thiazépine (1,2) yl-11) amino]-5 pentanoïque qui a la formule chimique (I) suivante :

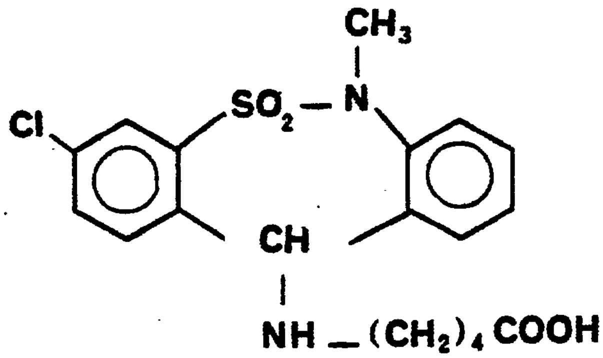

(I)

Ce composé possède un atome de carbone asymétrique et il existe donc sous forme d'isomères optiques qui font également, à ce titre, partie de l'invention.

Le nouveau dérivé de formule générale I est un composé amphotère donnant à la fois des sels d'addition avec des bases organiques ou minérales, et avec des acides minéraux ou organiques pharmaceutiquement acceptables. Tous ces sels sont inclus dans la présente invention. Parmi les bases utilisées pour l'obtention de ces sels, on peut citer les hydroxydes, les carbonates et les bicarbonates de sodium, potassium ou calcium, et parmi les acides utilisés, on peut citer les acides phosphorique, chlorhydrique, sulfurique, acétique, propionique, citrique, oxalique, benzoïque.

La présente invention a également pour objet le procédé de préparation du composé de formule I, caractérisé en ce que l'on condense un dérivé halogéné de formule générale II

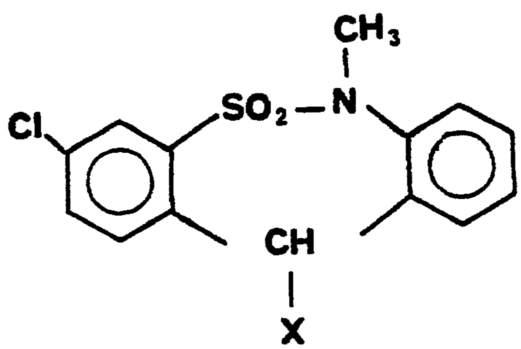

(II)

dans laquelle X représente un atome de chlore ou de brome,
avec un ester d'acide amino-6 pentanoïque de formule générale III

$NH_2 — (CH_2)_4COOR$ (III)

dans laquelle R représente un radical alkyle inférieur contenant de 1 à 4 atomes de carbone,

sous forme de base ou d'un de ses sels d'addition avec un acide minéral ou organique,
puis, l'on saponifie l'ester tricyclique ainsi obtenu pour former le dérivé de formule générale I.

La condensation des dérivés II et III s'effectue dans un solvant organique, adéquat tel que le nitrométhane, en présence d'un accepteur de l'hydracide formé au cours de la réaction et à une température comprise entre 20 et 100°C.

La saponification de l'ester tricyclique formé s'effectue en milieu alcalin hydroalcoolique.

Les dérivés halogénés de formule générale II sont décrits dans le brevet français N°1.566.191.

Le composé de formule I possède un profil antidépresseur très nettement supérieur à celui de l'acide [(chloro-3 méthyl-6 dioxo-5,5 dihydro-6,11 dibenzo (c,f) thiazépine (1,2) yl-11) amino]-7 heptanoïque qui est le composé le plus actif parmi ceux décrits dans le brevet français FR-A 2 104 728.

En effet, les essais pharmacologiques classiquement utilisés pour l'évaluation de l'activité antidépressive des nouveaux composés chimiques et qui sont exposés en particulier dans Enna S.J. et al. "Antidepressants: Neurochemical Behavioral, and Clinical Perspectives" Raven Press N.Y. 1981, p. 107-120 ont révélé que l'acide [(chloro-3 méthyl-6 dioxo-5,5 dihydro-6,11 dibenzo (c,f) thiazépine (1,2) yl-11) amino]-5 pentanoïque est doué d'une activité antidépressive très importante.

Les propriétés pharmacologiques du composé de l'invention permettent donc son application dans le traitement de la dépression.

L'invention s'étend aussi aux compositions pharmaceutiques renfermant comme principe actif le composé de formule I, l'un de ses isomères optiques ou l'un de ses sels d'addition avec une base ou un acide pharmaceutiquement compatible, en association avec un ou plusieurs excipients inertes, et non toxiques.

Les compositions pharmaceutiques ainsi obtenues sont présentées avantageusement sous des formes diverses telles que par exemple, comprimés, dragées, gélules, glossettes, ou autres préparations galéniques appropriées pour une administration sublinguale, suppositoires, solutions injectables ou buvables.

Les compositions pharmaceutiques selon l'invention peuvent en outre contenir un autre principe actif d'action complémentaire.

La posologie peut varier largement en fonction de l'âge ou du poids du patient, mais surtout de la voie d'administration et de la sévérité de l'affection.

La voie d'administration préférée est la voie orale ou parentérale. D'une manière générale, la posologie unitaire s'échelonnera entre 1 et 10 mg et la posologie journalière entre 1 et 50 mg.

Les exemples suivants, donnés à titre non limitatif, illustrent l'invention.

EXEMPLE 1 :

Acide [(chloro-3 méthyl-6 dioxo-5,5 dihydro-6,11 dibenzo (c,f) thiazépine (1,2) yl-11) amino]-5 pentanoïque

STADE A :

[(Chloro-3 méthyl-6 dioxo-5,5 dihydro-6,11 dibenzo (c,f) thiazépine (1,2) yl-11) amino]-5 pentanoate d'éthyle.

On porte 0,072 M de dichloro-3,11 méthyl-6 dioxo-5,5 dihydro-6,11 dibenzo (c,f) thiazépine (1,2) avec 0,072 M de chlorhydrate d'amino-5 pentanoate d'éthyle dans 215 ml de nitrométhane préalablement desséché, pendant 2 heures à 60°C sous agitation et en présence de 0,144 M de triéthylamine.

Après évaporation du solvant sous pression réduite, le résidu est repris par l'éther éthylique et l'eau. Après séparation de deux solvants, la phase organique est lavée à l'eau, et ensuite séchée sur sulfate de sodium anhydre. Après évaporation du solvant organique, on obtient sous forme de résidu huileux le [(chloro-3 méthyl-6 dioxo-5,5 dihydro-6,11 dibenzo (c,f) thiazépine (1,2) yl-11) amino] -5 pentanoate d'éthyle.

Rendement 82 %

Le spectre infra rouge de ce composé, obtenu avec une cuve de chlorure de sodium de 1 mm, présente une bande fine à 3.400 $cm^{-1}$, caractéristique de la fonction amine, et une bande à 1.720 $cm^{-1}$ qui correspond à la vibration de valence du carbonyle de la fonction ester.

STADE B :

Acide [(chloro-3 méthyl-6 dioxo-5,5 dihydro-6,11 dibenzo (c,f) thiazépine (1,2) yl-11) amino] -5 pentanoïque

0,039 M du produit obtenu au stade précédent sont dissous dans un mélange hydroalcoolique contenant 39 ml d'hydroxyde de sodium N et 80 ml d'éthanol. La solution est conservée 12 heures à la température ambiante, et ensuite, l'éthanol est évaporé sous vide. Le résidu est partagé entre eau et éther éthylique.

Après avoir ajouté une quantité suffisante d'acide chlorhydrique N pour acidifier légèrement la phase aqueuse (pH=6,3), un précipité huileux se forme; cette huile est extraite au dichlorométhane. La solution organique ainsi obtenue est lavée à l'eau et ensuite séchée sur sulfate de sodium anhydre. Après élimination du solvant sous vide, le résidu est repris par 100 ml d'acétonitrile, et cette dernière solution est refroidie à 0°C. Le précipité formé est isolé par filtration et ensuite recristallisé dans 180 ml d'acétonitrile. On obtient 0,020 M d'acide [(chloro-3 méthyl-6 dioxo-5,5 dihydro-6,11 dibenzo (c,f) thiazépine (1,2) yl-11) amino]-5 pentanoïque.

Rendement 49 %

Analyse élémentaire: ($C_{19}H_{21}ClN_2O_4S$)

| | C | H | N | Cl | S |
|---|---|---|---|---|---|
| % trouvé | 55,69 | 5,21 | 6,75 | 8,68 | 7,93 |
| % calculé | 55,80 | 5,17 | 6,85 | 8,67 | 7,84 |

Spectre de résonnance magnétique nucléaire du proton enregistré à 60 MHz en solution dans un mélange $CDCl_3$-D.M.S.O.

1,6 ppm,m,4H; 2,1 à 2,70 ppm,m,4H; 3,4 ppm,s,3H; 4,5 ppm, 2 protons échangeables, 5,0 ppm,s,1H; 7,2 à 8,0 ppm,m,7H.

Spectre de masse (Ionisation chimique $NH_3$ 0,3 Torr).

409 m/z $[M+1]^+$ (100%); 391 m/z (3%); 311 m/z (19,3%); 294 m/z (4,2 %); 292 m/z (21,1%); 232 m/z (9,6%); 230 m/z (10,2%); 118 m/z (9,9%).

ETUDE PHARMACOLOGIQUE

L'activité antidépressive du composé de formule I a été évaluée au moyen de différents essais in vivo et comparée à celle de l'acide [(chloro-3 méthyl-6 dioxo-5,5 dihydro-6,11 (c,f) thiazépine (1,2) yl-11) amino]-7 heptanoïque (ATAH).

EXEMPLE 2 :

Effets sur la ptose induite par la réserpine chez la souris

L'expérience est réalisée sur des groupes de 20 souris NMRI (IFFA-CREDO) mâles, de poids moyen de 20 ± 2 g. Le composé de formule I et l'ATAH, à la dose de 25 $mg.kg^{-1}$, ou l'eau pour préparations injectables, sont administrés par voie intrapéritonéale, 1 heure avant l'injection intraveineuse d'une dose de 2 $mg.kg^{-1}$ de réserpine, selon une méthode décrite par GOURET C. et coll., dans J. Pharmacol., 1977, 8 (3), p. 333-350.

Une heure après l'injection de réserpine, les animaux sont déposés sur une plateforme carrée en bois de 5 cm de côté, pendant 20 secondes. Le ptosis est considéré comme positif si l'animal garde les yeux fermés pendant les 20 secondes qui suivent son dépôt sur la plateforme. En revanche, l'animal est considéré comme protégé, s'il n'a pas présenté de fermeture complète des yeux dans les 20 secondes qui suivent le moment où il est déposé sur la plateforme.

L'ATAH, à la dose de 25 $mg.kg^{-1}$ I.P., exerce un antagonisme faible vis-à-vis du ptosis induit par 2 $mg.kg^{-1}$ I.V. de réserpine, puisque 32 % seulement des animaux sont protégés. Le composé de formule I, à la même dose, est beaucoup plus actif et protège 53 % des animaux.

EXEMPLE 3 :

Effets sur l'hypothermie induite par la réserpine chez la souris

L'étude est réalisée sur des groupes expérimentaux constitués de 10 souris NMRI (IFFA-CREDO) à jeun depuis 18 heures. Les groupes "essais" de souris sont traités par administration intrapéritonéale de 50 $mg.kg^{-1}$ de ATAH ou de composé de formule I. Le groupe "témoins" reçoit le même volume de sérum physiologique. Une heure après, tous les animaux reçoivent par voie intrapéritonéale 2 $mg.kg^{-1}$ de réserpine. L'hypothermie est évaluée 4 heures après l'injection de réserpine par mesure de la température rectale au moyen d'un thermomètre électrique.

Comme il est indiqué sur le tableau I, les deux produits exercent un antagonisme vis-à-vis de l'hypothermie induite par la réserpine. Cet antagonisme se traduit dans le cas de l'ATAH par une élévation de température de +10 % par rapport à celle du lot témoin. Dans le cas du composé de formule I, l'activité antagoniste est très supérieure, puisque la dose de 50 $mg.kg^{-1}$ I.P. produit une élévation de température de l'ordre de +25 %.

Tableau I

| Traitement I.P. | Températures finales | |
|---|---|---|
| | Moyenne ± e.t. | % Variation |
| Témoins eau + Réserpine 2 $mg.kg^{-1}$ | 24,7 ± 0,88 | |
| ATAH 50 $mg.kg^{-1}$ + Réserpine 2 $mg.kg^{-1}$ | 27,1 ± 1,40 | + 10% |
| Composé de formule I 50 $mg.kg^{-1}$ + Réserpine 2 $mg.kg^{-1}$ | 30,9 ± 3,7 | + 25% |

e.t = écart-type

EXEMPLE 3 :

Effets sur la ptose induite par la tétrabénazine chez la souris

Des souris mâles de souche CD (Charles River) d'un poids moyen de 23 g sont réparties en lots de 12 animaux. Le composé de formule I ou le ATAH ou l'eau pour préparations injectables (témoins) sont administrés par voie intrapéritonéale, 15 mn avant l'injection de 20 $mg/kg^{-1}$ de tétrabénazine par la même voie. La dose des produits soumise à l'essai est 25 $mg.kg^{-1}$. Les animaux sont observés individuellement 5, 25, 35, et 55 minutes après l'injection de tétrabénazine et l'intensité du ptosis est appréciée selon une échelle allant de 0 à 4.

Comme les résultats rapportés au Tableau II le démontrent, à la dose de 25 $mg.kg^{-1}$, le composé de formule I a une activité en intensité et en durée beaucoup plus importante que l'ATAH.

Tableau II

| Traitement | Valeur moyenne du score d'intensité du Ptosis ± e.t. | | | |
|---|---|---|---|---|
| | 5 minutes | 25 minutes | 35 minutes | 55 minutes |
| Témoins m | 1,33 ± 0,65 | 2,58 ± 0,51 | 2,83 ± 0,58 | 3,08 ± 0,51 |
| Composé de form. I m | 0,42 ± 0,51 | 1,75 ± 0,45 | 1,83 ± 0,39 | 2,42 ± 0,67 |
| % témoins | –69 | –32 | –35 | –22 |
| ATAH + tétrabénazine m | 0,50 ± 0,52 | 1,92 ± 0,51 | 2,25 ± 0,45 | 2,58 ± 0,51 |
| % témoins | –62,5 | –26 | –21 | NS |

m = moyenne des scores observés
% témoins = % variation du nombre total des scores moyens par rapport aux témoins
NS = non significatif
e.t. = écart-type

EXEMPLE 5 :

Effets sur l'agressivité de souris isolées

Les souris CD (Charles RIVER) mâles utilisées dans cette étude, sont maintenues en cages individuelles aux parois peintes en noir et de petite taille (18 x 10 x 10 cm), à raison d'une souris par cage, depuis plusieurs mois, à une température constante de 21°C et sous un cycle lumière-obscurité de 12 heures. Leur poids moyen, au moment de l'étude, est de 45 g.

Chaque lot expérimental comporte 10 couples de souris sélectionnées au préalable pour leur agressivité. Le test d'agressivité est effectué sur les animaux non à jeun. Il consiste à placer la souris "intruse" du couple, dans la boîte contenant l'autre souris du couple, pendant une période de 3 minutes. On note, pour chaque lot expérimental de 10 couples, le pourcentage de couples "protégés", c'est-à-dire de couples n'ayant effectué aucune attaque pendant les 3 minutes du test.

Dans l'étude présente, les doses de 10 et 20 $mg.kg^{-1}$ de ATAH et du composé de formule I, ont été soumises à l'essai. Le produit est administré par voie intrapéritonéale, 30 minutes avant le test.

Comme il est indiqué sur le tableau III, seule la dose de 20 $mg.kg^{-1}$ IP de ATAH ou de composé de formule I exerce une activité anti-agressive nette sur ce test, avec respectivement 40 et 60 % de couples

protégés (0 % dans le lot témoin). Sur ce tableau, la mention S représente des variations statistiquement significatives.

L'activité anti-agressive du composé de formule I est très supérieure à celle du ATAH, sur ce modèle.

Tableau III

| Traitement | Nombre d'attaques moyenne ± e.t. | % des couples protégés (sans attaque) |
|---|---|---|
| Témoins eau | 6,7 ± 4,7 | 10% |
| ATAH 10 $mg.kg^{-1}$ | 7,2 ± 4,6 | 10% |
| Témoins eau | 5,4 ± 2,8 | 10% |
| Composé de formule I 10 $mg.kg^{-1}$ | 7,2 ± 5,9 | 20% |
| Témoins eau | 8,3 ± 6,0 | 0% |
| ATAH 20 $mg.kg^{-1}$ | 3,7 ± 4,9 | 40% S |
| Composé de formule I 20 $mg.kg^{-1}$ | 3,9 ± 6,2 | 60% S |

e.t = écart-type

PREPARATION PHARMACEUTIQUE

EXEMPLE 6 :

Gélules dosées à 0,005 g d'acide [(chloro-3 méthyl-6 dioxo- 5,5 dihydro-6,11 dibenzo (c,f) thiazépine (1,2) yl-11 amino]-5 pentanoïque

| | |
|---|---|
| Acide [(chloro-3 méthyl-6 dioxo-5,5 dihydro-6,11 dibenzo (c,f) thiazépine (1,2) yl-11) amino]-5 pentanoïque | 0,0050 g |
| Amidon de maïs | 0,0320 g |
| Cellulose microcristalline | 0,0262 g |
| Lactose | 0,0720 g |
| Silice colloïdale | 0,0030 g |
| Stérate de magnésium | 0,0015 g |
| Talc pour une gélule blanche taille N° 3 | 0,0030 g |

**Revendications pour les Etats contractants: BE, CH, DE, FR, GB, IT, LI, LU, NL, SE**

1. Composé de formule I

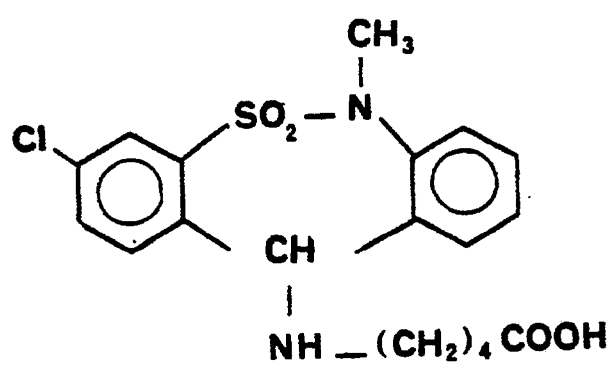

(I)

sous forme racémique ou d'isomères optiques et ses sels d'addition à une base organique ou minérale ou à un acide minéral ou organique pharmaceutiquement acceptable.

2. Procédé de préparation du composé de formule I, caractérisé en ce que l'on condense un dérivé halogéné de formule générale II

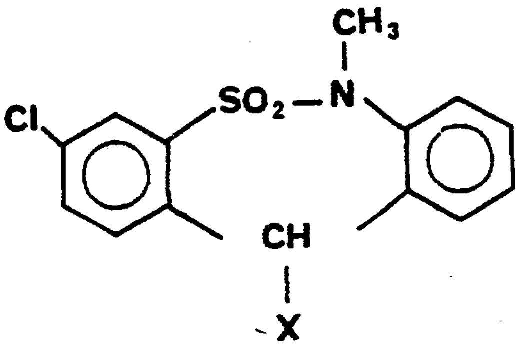

(II)

dans laquelle X représente un atome de chlore, ou de brome, avec un ester d'acide amino-5 pentanoïque de formule générale III

$NH_2 — (CH_2)_4COOR$ (III)

dans laquelle R représente un radical alkyle inférieur contenant de 1 à 4 atomes de carbone, sous forme de base ou d'un de ses sels d'addition avec un acide minéral ou organique, puis l'on saponifie l'ester tricyclique ainsi obtenu pour former le dérivé de formule générale I.

3. Composition pharmaceutique contenant comme principe actif le composé selon la revendication 1, en association ou en mélange avec un excipient ou un véhicule inerte non toxique, pharmaceutiquement acceptable.

4. Composition pharmaceutique selon la revendication 3 renfermant le principe actif à la dose de 1 à 10 mg.

5. Composition pharmaceutique selon les revendications 3 à 4 utilisable pour le traitement de la dépression.

**Revendication pour l'Etat contractant: AT**

1. Procédé de préparation du composé de formule I

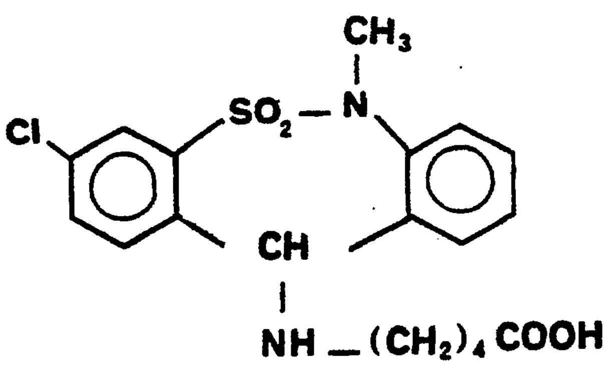

(I)

sous forme racémique ou d'isomères optiques et ses sels d'addition à une base organique ou minérale ou à un acide minéral ou organique pharmaceutiquement acceptable, caractérisé en ce que l'on condense un dérivé halogéné de formule générale II

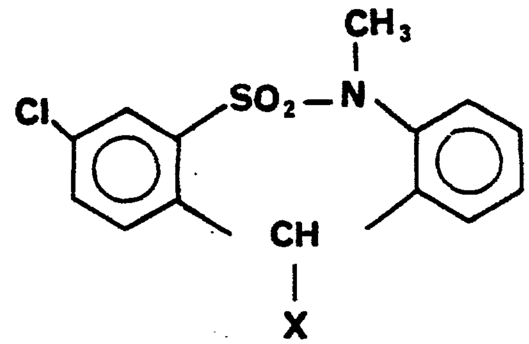

(II)

dans laquelle X représente un atome de chlore, ou de brome, avec un ester d'acide amino-5 pentanoïque de formule générale III

$NH_2 — (CH_2)_4COOR$ (III)

dans laquelle R représente un radical alkyle inférieur contenant de 1 à 4 atomes de carbone, sous forme de base ou d'un de ses sels d'addition avec un acide minéral ou organique, puis l'on saponifie l'ester tricyclique ainsi obtenu pour former le dérivé de formule générale I.

**Claims for the Contracting States: BE, CH, DE, FR, GB, IT, LI, LU, NL, SE**

1. Compound of the formula I

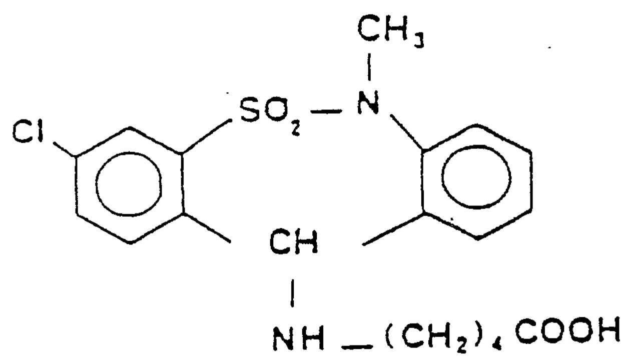

(I)

in its racemic form or optical isomers and addition salts thereof with a pharmaceutically acceptable organic or mineral base or mineral or organic acid.

2. Process for the preparation of the compound of formula I, which comprises condensing a halogenated derivative of the general formula II

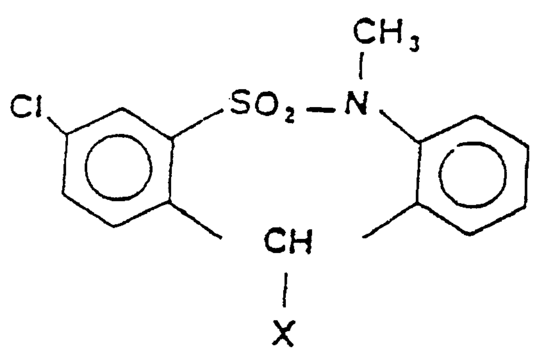

(II)

in which X represents a chlorine or bromine atom, with a 5-amino pentanoic acid ester or the general formula III

$NH_2–(CH_2)_4COOR$ (III)

in which R represents a lower alkyl radical containing 1 to 4 carbon atoms, in the form of a base or one of its addition salts with a mineral or organic acid, and then saponifying the tricyclic ester so obtained to yield the derivative of the general formula I.

3. Pharmaceutical composition containing the compound according to claim 1 as active principle, in association or in admixture with a pharmaceutically acceptable, non toxic, inert excipient or vehicle.

4. Pharmaceutical composition acccrding to claim 3 containing the active principle at the dose of 1 to 10 mg.

5. Pharmaceutical composition according to claim 3 and 4 used in the treatment of depression.

**Claim for the Contracting State: AT**

1. Process for the preparation of the compound of the formula I

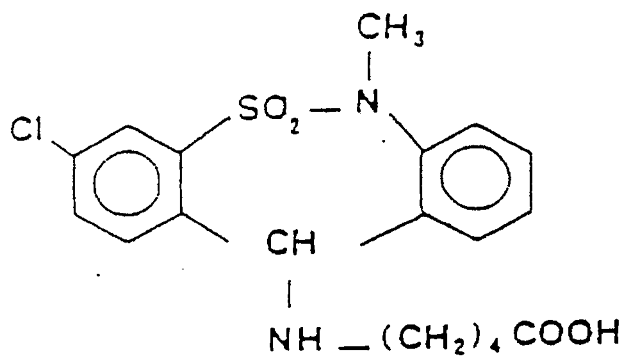

(I)

in its racemic form or optical isomers and addition salts thereof with a pharmaceutically acceptable organic or mineral base or mineral or organic acid, which comprises condensing a halogenated derivative of the general formula II

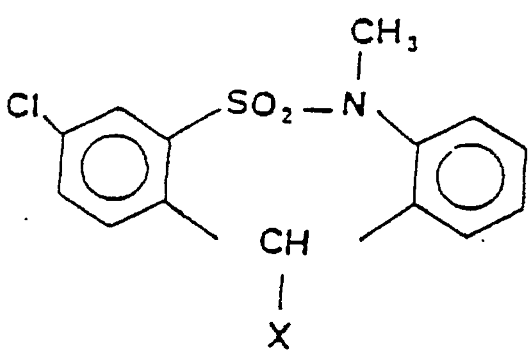

(II)

in which X represents a chlorine or bromine atom, with a 5-amino pentanoic acid ester of the general formula III

$NH_2-(CH_2)_4COOR$ (III)

in which R represents a lower alkyl radical containing 1 to 4 carbon atoms, in the form of a base or one of its addition salts with a mineral or organic acid, and then saponifying the tricyclic ester so obtained to yield the derivative of the general formula I.

**Patentansprüche für die Vertragsstaaten: BE, CH, DE, FR, GB, IT, LI, LU, NL, SE**

1. Verbindung der Formel I

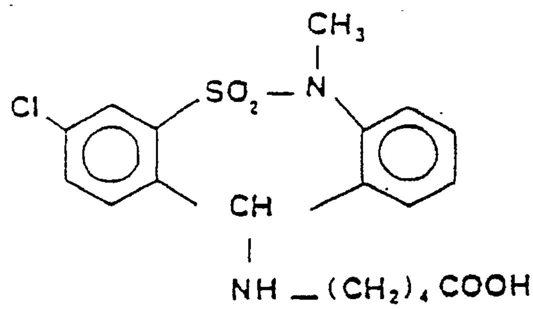

(I)

in Form des Racemats oder der optischen Isomeren und ihre Additionssalze mit einer organischen oder anorganischen Base oder mit einer pharmazeutisch annehmbaren, anorganischen oder organischen Säure.

2. Verfahren zur Herstellung der Verbindung der Formel I, dadurch gekennzeichnet, daß man ein Halogenderivat der allgemeinen Formel II

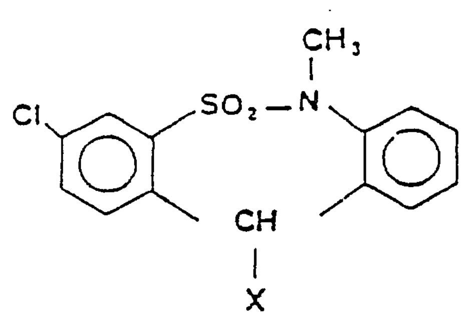

(II)

in der X ein Chloratom oder ein Bromatom bedeutet, mit einem Ester der 5-Amino-pentansäure der allgemeinen Formel III

$NH_2-(CH_2)_4COOR$ (III)

in der R eine niedrigmolekulare Alkylgruppe mit 1 bis 4 Kohlenstoffatomen bedeutet, in Form der Base oder eines seiner Additionssalze mit einer anorganischen oder organischen Säure kondensiert und dann den in dieser Weise erhaltenen tricyclischen Ester zur Bildung des Derivats der allgemeinen Formel I verseift.

3. Pharmazeutische Zubereitung enthaltend als Wirkstoff die Verbindung nach Anspruch 1, in Kombination oder in Mischung mit einem inerten, nicht-toxischen pharmazeutisch annehmbaren Bindemittel oder Trägermaterial.

4. Pharmazeutische Zubereitung nach Anspruch 3, enthaltend den Wirkstoff in einer Dosis von 1 bis 10 mg.

5. Pharmazeutische Zubereitung nach den Ansprüchen 3 bis 4 zur Verwendung bei der Behandlung von Depresssionen.

**Patentanspruch für den Vertragsstaat: AT**

1. Verfahren zur Herstellung der Verbindung der Formel I

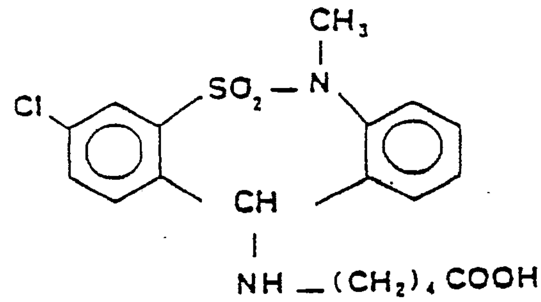

(I)

in Form des Racemats oder der optischen Isomeren und ihrer Additionssalze mit einer organischen oder anorganischen Base oder einer pharmazeutisch annehmbaren, anorganischen oder organischen Säure, dadurch gekennzeichnet, daß man ein Halogenderivat der allgemeinen Formel II

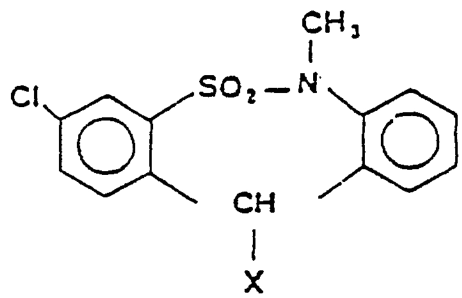

(II)

in der X ein Chloratom oder ein Bromatom bedeutet, mit einem Ester der 5-Amino-pentansäure der allgemeinen Formel III

$NH_2-(CH_2)_4COOR$ (III)

in der R eine niedrigmolekulare Alkylgruppe mit 1 bis 4 Kohlenstoffatomen bedeutet, in Form der Base oder eines seiner Additionssalze mit einer anorganischen oder organischen Säure kondensiert und dann den in dieser Weise erhaltenen tricyclischen Ester zur Bildung des Derivats der allgemeinen Formel I verseift.